(19) Europäisches Patentamt
European Patent Office
Office européen des brevets

(11) **EP 3 494 389 B1**

(12) **EUROPÄISCHE PATENTSCHRIFT**

(45) Veröffentlichungstag und Bekanntmachung des Hinweises auf die Patenterteilung:
**21.12.2022 Patentblatt 2022/51**

(21) Anmeldenummer: **17746480.7**

(22) Anmeldetag: **01.08.2017**

(51) Internationale Patentklassifikation (IPC):
*G01N 22/04* (2006.01)   *A61F 13/15* (2006.01)

(52) Gemeinsame Patentklassifikation (CPC):
**G01N 22/04; A61F 13/15772**

(86) Internationale Anmeldenummer:
**PCT/EP2017/069459**

(87) Internationale Veröffentlichungsnummer:
**WO 2018/024735 (08.02.2018 Gazette 2018/06)**

(54) **VORRICHTUNG UND VERFAHREN ZUR MESSUNG VON ABSORBIERENDEN HYGIENEARTIKELN**

DEVICE AND METHOD FOR MEASURING ABSORBENT HYGIENE ARTICLES

DISPOSITIF ET PROCÉDÉ DE MESURE D'ARTICLES D'HYGIÈNE ABSORBANTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.08.2016 DE 102016114287**

(43) Veröffentlichungstag der Anmeldung:
**12.06.2019 Patentblatt 2019/24**

(73) Patentinhaber: **TEWS Elektronik GmbH & Co. KG 22459 Hamburg (DE)**

(72) Erfinder: **SCHLEMM, Udo 22607 Hamburg (DE)**

(74) Vertreter: **Hauck Patentanwaltspartnerschaft mbB Postfach 11 31 53 20431 Hamburg (DE)**

(56) Entgegenhaltungen:
EP-A2- 1 933 132        EP-A2- 2 207 027
US-A1- 2016 051 414

**EP 3 494 389 B1**

**Beschreibung**

Die vorliegende Erfindung betrifft eine Vorrichtung und ein Verfahren zur Messung von absorbierenden Hygieneartikeln, insbesondere zur Messung bei absorbierenden Hygieneartikeln während ihrer Herstellung, bei der auf eine fortlaufende Bahn voneinander beabstandete Saugkörper aufgebracht werden.

Aus EP 1 327 876 B1 ist ein Verfahren zum Erkennen von Fremdkörpern in kontinuierlichen Masseströmen aus faserförmigem, strangförmigem oder schüttgutartigem Material mit Hilfe eines Mikrowellenresonators bekannt geworden. Der Massestrom wird durch ein Feld des Mikrowellenresonators geführt, bei dem die durch das Material bewirkte Änderung (A) der Resonanzfrequenz und die Änderung (B) der Breite der Resonanzkurve des Mikrowellenresonators bestimmt wird. Das Verhältnis der Änderungen wird mit entsprechenden Mittelwerten verglichen und die Anwesenheit eines Fremdkörpers wird gemeldet, wenn das Verhältnis der Änderungen von dem Mittelwert um mehr als einen vorgegebenen Wert abweicht.

Aus WO 2014/170796 A1 ist ein Verfahren zur Herstellung von absorbierenden Hygieneartikeln bekannt. Der absorbierende Hygieneartikel, beispielsweise in Form einer Windel, weist ein absorbierendes Pad als Saugkörper auf, das wiederum eine oder mehrere absorbierende Materialen besitzt. Die absorbierenden Pads werden entlang einer Bahn eines durchlässigen Materials aufgebracht, wobei ein Mikrowellenresonator vorgesehen ist, der ein Gewichtsprofil oder ein Dichteprofil des mindestens einen absorbierenden Materials erstellt. Für die Messposition des Mikrowellenresonators werden unterschiedliche Positionen an der Windelmaschine vorgeschlagen. Besondere Probleme bei der Auswertung der Daten ergeben sich aus dem Umstand, dass bei der Herstellung von Windeln die Windelkerne mit geringem Abstand auf die endlose Bahn von PE-Außenhülle und permeabler Deckschicht aufgebracht werden. In diesem Sinne gibt es keinen Leerbetrieb des Mikrowellenresonators, der für einen Abgleich von Leerwerten genutzt werden könnte.

Aus EP 1 933 132 A2 ist ein Verfahren zur Bestimmung der Feuchte einer laufenden Materialbahn bekannt, die mittels eines Mikrowellenresonators vermessen wird.

Aus US 2016/051414 A1 ist ein Verfahren zur Messung von absorbierenden Hygieneartikeln bekannt, bei dem auf eine fortlaufende Bahn voneinander beabstandete Saugkörper aufgebracht werden, deren Dichte mit einem Mikrowellenresonator erfasst wird, wobei die Bahn durch die Mikrowellenresonatoren transportiert wird und fortlaufend Mikrowellenmessgrößen erfasst werden.

Der Erfindung liegt die Aufgabe zugrunde, eine Vorrichtung und ein Verfahren zur Messung von absorbierenden Hygieneartikeln mit Hilfe eines Mikrowellenresonators bereitzustellen.

Erfindungsgemäß wird die Aufgabe durch eine Vorrichtung mit den Merkmalen nach Anspruch 1 gelöst. Vorteilhafte Ausgestaltungen bilden den Gegenstand der Unteransprüche.

Die erfindungsgemäße Vorrichtung dient zur Messung von absorbierenden Hygieneartikeln, wobei bei dem Verfahren auf eine fortlaufende Bahn voneinander beabstandete Saugkörper aufgebracht werden. Feuchte und/oder Dichte der Saugkörper werden in zwei oder mehr Mikrowellenresonatoren mit Hilfe einer Resonanzfrequenzverschiebung (A) und einer Resonanzfrequenzverbreiterung (B) erfasst. Die Bahn wird durch die Mikrowellenresonatoren transportiert und fortlaufend werden jeweils zwei Mikrowellenwerte bestimmt.

Gerade bei der Herstellung von absorbierenden Hygieneartikeln, insbesondere von Windeln, kann das Problem auftreten, dass die fortlaufend zu vermessende Bahn für einen Mikrowellenresonator zu breit, bezogen auf die Transportrichtung, ist. Dieses Problem wird durch die Verwendung von mindestens zwei Mikrowellenresonatoren gelöst, die bezogen auf eine Transportrichtung der Bahn zueinander in Quer- und in Transportrichtung versetzt angeordnet sind, um die Bahn in ihrer gesamten Breite zu vermessen. Bezogen auf die Transportrichtung sind die zwei oder mehr Mikrowellenresonatoren in Querrichtung zueinander versetzt angeordnet, um die Bahn in ihrer gesamten Breite vermessen zu können. Hier ist es erforderlich, die quer zueinander versetzten Mikrowellenresonatoren auch in Transportrichtung zueinander versetzt anzuordnen, da diese in der Regel in einem zentralen Bereich eine homogene Feldverteilung besitzen. Durch den Versatz der Mikrowellenresonatoren in Längsrichtung können diese so überlappend zueinander angeordnet werden, dass die Bahn in ihrer gesamten Breite mit einer homogenen Feldverteilung vermessen wird.

Erfindungsgemäß besitzt jeder der Mikrowellenresonatoren einen Messbereich mit einem homogenen Feldverlauf, wobei die mehreren Mikrowellenresonatoren derart quer zur Transportrichtung positioniert sind, dass das gesamte Band von Messbereichen mit homogenem Feldverlauf überdeckt wird. Ein homogener Feldverlauf hilft, zuverlässige und genaue Messergebnisse zu erzielen. Durch den homogenen Feldverlauf ist es auch möglich, die aufgenommenen Messwerte in Querrichtung der Bahn zu mitteln. Mehrere Mikrowellenresonatoren werden derart hintereinander angeordnet, dass das Band in seiner Breite insgesamt mit einem Messbereich mit homogenem Feldverlauf überdeckt ist.

In einer bevorzugten Weiterentwicklung des erfindungsgemäßen Messverfahrens bildet jeder Mikrowellenresonator einen Mittelwert seiner Messwerte in Querrichtung, bezogen auf die Transportrichtung der Bahn. Die gemittelten Messwerte der mehreren Mikrowellenresonatoren werden um einen Versatz der Mikrowellenresonatoren in Transportrichtung korrigiert, um einen Gesamtmittelwert für die gesamte Breite der Bahn zu bestimmen. Bei der Korrektur um den Versatz der Mikrowellenresonatoren wird berücksichtigt, dass aufgrund des räumlichen Versatzes der Mikrowellenresonatoren zueinander und der Transportgeschwindigkeit der Bahn die gemittelten Messwerte mit einem zeitlichen Versatz zueinander addiert werden müssen, um einen Mittelwert für die gesamte Breite der Bahn zu erhalten.

Die erfindungsgemäße Aufgabe wird ebenfalls durch ein Verfahren mit den Merkmalen aus Anspruch 6 gelöst. Vorteilhafte Ausgestaltungen des Verfahrens bilden den Gegenstand der Unteransprüche.

Bei dem erfindungsgemäßen Verfahren nach Anspruch 6 werden mindestens zwei Mikrowellenresonatoren fortlaufend zur Messung eingesetzt, wobei die Mikrowellenresonatoren, bezogen auf die Transportrichtung der Bahn, zueinander in Quer- und in Transportrichtung versetzt zueinander angeordnet sind. Die Bahn wird durch die mindestens zwei Mikrowellenresonatoren in ihrer vollen Breite vermessen.

Bevorzugt mittelt jeder der Mikrowellenresonatoren seine Messwerte in Querrichtung, so dass die gemittelten Messwerte in Querrichtung um den Versatz der Mikrowellenresonatoren in Transportrichtung korrigiert werden. Auf diese Art und Weise wird sichergestellt, dass stets eine Auswertung der Bahn in einem sich über die gesamte Breite erstreckenden Messbereich erfolgt.

Zur Bestimmung der Masse und/oder Feuchte der Saugkörper wird ein Ansatz gewählt, der die aufsummierten Messwerte der Mikrowellengrößen bestimmt. Bevorzugt werden, um unabhängig von der Transportgeschwindigkeit zu sein, die aufsummierten Messwerte der beiden Messgrößen durch die Anzahl der Summanden geteilt. Der so bestimmte mittlere Summenwert hängt nur von der Länge des Saugkörpers in Transportrichtung, nicht aber von der Transportgeschwindigkeit ab.

In einer bevorzugten Ausgestaltung des erfindungsgemäßen Verfahrens wird ebenfalls die Masse eine Saugkörpers über ein Aufsummieren der Messwerte bestimmt. Hierbei wird bevorzugt jeder einzelne Messwert aufsummiert und in Relation mit der zu bestimmenden Gesamtmasse gesetzt. Die aufsummierten Messwerte werden durch die Anzahl ihrer Summanden geteilt.

Das erfindungsgemäße Verfahren wird nachfolgend an einem Ausführungsbeispiel näher erläutert. Es zeigen:

Fig. 1    den grundsätzlichen Aufbau einer Windelmaschine sowie mögliche Positionen für einen Mikrowellenresonator,

Fig. 2    Signale eines Windelstrangs, bei dem der Abstand der Windelkerne kleiner als ein Sensordurchmesser ist,

Fig. 3    Signale eines Windelstrangs, bei dem der Abstand der Windelkerne größer als der Windeldurchmesser ist,

Fig. 4    eine schematische Ansicht des Feldverlaufs in einem Gabelresonator in Längsrichtung und

Fig. 5    eine Anordnung von zwei Gabelsensoren bei einem bahnförmigen Messgut.

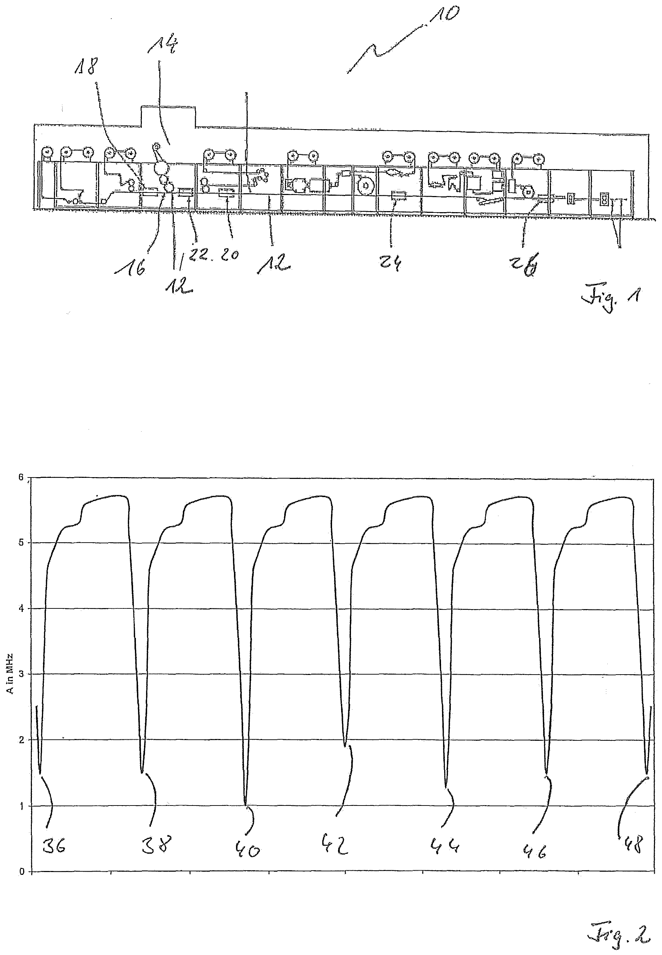

Fig. 1 zeigt in einer schematischen Ansicht den Gesamtaufbau einer Windelmaschine 10. Die Maschine 10 besitzt eine fortlaufende Bahn 12 aus einer PE-Außenhülle, die kontinuierlich mit großer Geschwindigkeit durch die Maschine läuft, um eine Windel herzustellen. In periodischen Abständen werden Windelkerne in einem Abschnitt 14 der Maschine aufgebracht. Die aufgebrachten Windelkerne bestehen aus absorbierenden Pads und werden auf der durchlaufenden Bahn 12 positioniert. Bei den schematisch dargestellten Detektionseinrichtungen 18 und 20 handelt es sich um herkömmlich arbeitende Detektionseinrichtungen, die beispielsweise die Bahn und den aufgebrachten Windelkern optisch überwachen. Ein Mikrowellenresonator 22, 24, 26 ist entlang des Produktionsprozesses positioniert, um immer wieder eine Masse und Feuchtemessung an den Windeln und der Bahn 12 durchzuführen. Je nach Position des Mikrowellenresonators entlang der Bahn 12 können die Mikrowellenmesswerte für Masse und Feuchte in unterschiedlichen Stadien der Herstellung gemessen und überwacht werden.

Eine Einweg- oder Wegwerfwindel besteht aus einer Außenhülle aus Polyethylen (PE) und einem Saugkörper, der in der Regel aus einem Zellstoffmaterial besteht, das mit einem Superabsorber (Polymer-Salze) angereichert ist. Durch den Saugkörper kann die Flüssigkeitsmenge um das Vielfache des Eigenvolumens gebunden werden, und auch bei Druckausübung kann die Flüssigkeit gehalten werden.

Bei der Herstellung von Windeln wird in der Windelmaschine 10 eine endlose Bahn 12 der PE-Außenhülle mit einer permeablen Deckschicht in periodischen Abständen mit den Saugkörpern versehen. Die Saugkörper bestehen dabei aus einer Mischung von Zellulose und pulverförmigem Superabsorber (SAP = super absorbent polymere material).

Zur Qualitätskontrolle in der Produktion ist es wünschenswert, die Gesamtmasse und/oder ein Dichteprofil der Windelkerne mit Mikrowellenresonatoren zu messen. Aus WO 2014/170796 A1 sind eine Reihe von Vorschlägen zur Positionierung der Mikrowellenresonatoren in einer Windelmaschine bekannt. Ein Ansatz zur Auswertung der Messwerte um zuverlässige und exakte Ergebnisse zu erzielen, ist nicht bekannt.

Mit einem Mikrowellenresonator wird eine Verschiebung der Resonanzfrequenz A und eine Verbreiterung der Resonanzkurve B als Folge der dielektrischen Eigenschaften der zu untersuchenden Probe gemessen. Die Messwerte werden als Differenz der Werte des gefüllten und des leeren Resonators gebildet. Die Resonanzfrequenzverschiebung A in Hz lautet:

$$A = f_0 - f_m,$$

wobei $f_0$ die Resonanzfrequenz des leeren Resonators in Hz und $f_m$ die Resonanzfrequenz des gefüllten Resonators in Hz angibt. Für die Verbreiterung der Resonanzkurve wird auf die Vergrößerung der Halbwertsbreite der Resonanz B in Hz abgestellt. Hier gilt:

$$B = w_m - w_0,$$

wobei $w_0$ die Halbwertsbreite der Resonanz des leeren Resonators in Hz und $w_m$ die Halbwertsbreite der Resonanz des gefüllten Resonators in Hz bezeichnet.

Aus dem vorstehenden Ansatz wird deutlich, dass zu jeder Messung Informationen über die Resonanzparameter des leeren Resonators gehören. Die Leerresonanzwerte verändern sich bei Temperaturänderung und bei Verschmutzung des Resonators. Um den Einfluss der Leerresonanzwerte auf die Messwerte zu unterdrücken, werden in der Praxis folgende Maßnahmen getroffen:

- Bei Labormessungen erfolgt für jede Messwertaufnahme eine Messung der aktuellen Leerresonanzwerte.
- Bei Prozessmessungen, bei denen die Leerresonanzwerte nur selten gemessen werden können, wird der Sensor gekapselt und auf eine konstante Temperatur geregelt. Zusätzlich erfolgt regelmäßig eine Reinigung des Sensors mittels Druckluft.

Im Fall von portionierten Einheiten, die in einem endlosen, nicht metallischen Trägermaterial eingebettet sind, ist als Vorgehen in DE 10 2009 004 457 A1 der Fall beschrieben, bei dem die Abstände der portionierten Einheiten in dem Trägermaterial größer als der Durchmesser des eingesetzten Mikrowellenresonators ist. Somit ist sichergestellt, dass der Sensor zwischen den portionierten Einheiten nur das Trägermaterial erfasst. Die bekannte Auswertung setzt voraus, dass sich in periodischen Abständen nur das Trägermaterial, also die Bahn ohne portionierte Einheit in dem Messbereich befindet. Dies bedeutet, der Abstand der portionierten Einheit muss größer als das verwendete Messfeld sein.

Bei der Anwendung in der Windelmaschine ist der Abstand zwischen zwei Windelkernen häufig kleiner als der Durchmesser des Sensors. Es befindet sich also zu keinem Zeitpunkt nur das Trägermaterial allein in dem Sensor. Dies bedeutet, dass der Sensor stets zu einem gewissen Anteil Messsignale des Windelpakets enthält. Gleichwohl konnte festgestellt werden, dass bei der Anwendung von Windelkernen entlang der Bahn periodische Signalminima zwischen den Kernen auftreten. Die Erfindung beruht auf der Erkenntnis, dass bei Detektion dieser Minima anstatt eines Leerabgleichs die Signale aufgrund der Differenz zwischen den Messwerten A und B und den Messwerten der jeweiligen lokalen Minima $A_{min}$ und $B_{min}$ berechnet werden können. Somit ist es möglich, ohne Leerabgleich (wie er aus dem Stand der Technik bekannt ist) sämtliche Temperatureinflüsse auf den Sensor sowie Signalschwankungen durch Verschmutzung zu kompensieren.

Der erfindungsgemäße Auswertealgorithmus für die Mikrowellenresonatoren ist im besonderen Maße für Abstände zwischen Windelkernen geeignet, die kleiner als der Messbereich der Mikrowellenresonatoren ist. Selbstverständlich kann die erfindungsgemäße Auswertung über die lokalen Minima-Werte und deren gleitenden Mittelwert auch eingesetzt werden, wenn aufgrund einer besonderen Konfiguration der Windelmaschine die Abstände zwischen den Windelkernen größer als das verwendete Messfeld ist. Das erfindungsgemäße Verfahren ist somit universell und unabhängig von dem Abstand der Windelkerne einsetzbar.

Ein weiterer Vorteil bei Verwendung der Minimumsdetektion liegt darin, dass bei den bekannten Auswerteverfahren nach dem Stand der Technik beim Start des Messvorgangs der Windelstrang in eine vordefinierte Position relativ zu dem Sensor gefahren werden muss, d. h., der Bereich zwischen zwei Windelkernen muss sich beispielsweise im Sensor befinden. In dieser Position wird der erste Leerabgleich durchgeführt, der dann als Basis für die folgenden periodischen Leerabgleiche gilt. Diese Prozedur entfällt bei Verwendung der Minimumsdetektion, da bei Verwendung dieses Verfahrens kein Sensor - Leerabgleich benötigt wird.

Auch das aus EP 1 467 191 A1 bekannte Verfahren zur Messung von Kapseln/Tabletten setzt stets voraus, dass zwischen zwei zu messenden Tabletten das Transportband ohne Kapsel oder Tablette zur Messung eines Leerabgleichs vorliegt.

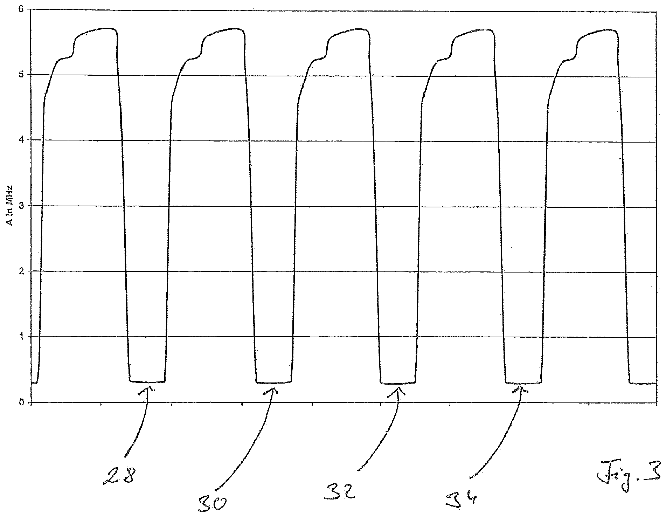

Fig. 3 zeigt den Signalverlauf der Resonanzfrequenzverschiebung A, in dem der Abstand zwischen zwei Windelkernen in Transportrichtung größer als die Ausdehnung des Messbereichs in Transportrichtung ist. Bei dieser Messsituation treten in periodischen Abständen zwischen den Windelkernen Minimumswerte 28, 30, 32, 34 auf, in denen sich nur die Bahn 12 in dem Messbereich befindet. Deutlich zu erkennen ist, dass die Messwerte für eine Konfiguration, bei der die Bahn ohne Windelkern durch den Mikrowellenresonator läuft, annähernd konstant sind und sich somit gut für einen Leerabgleich der Signalwerte ändern.

Das Verhalten der Messgrößen, insbesondere im Minimumsbereich, ändert sich deutlich in Fig. 2. Hier ist der Abstand zwischen zwei Windelkernen kleiner als der Messbereich eines Mikrowellenresonators. In Fig. 2 zu erkennen ist, dass die lokalen Minimumswerte 36 bis 50 ganz erheblich voneinander schwanken. So sind beispielsweise die lokalen Minimumswerte 40 und 42 fast um ein MHz voneinander verschieden. Deutlich wird das Ausmaß der Schwankung, wenn man hier berücksichtigt, dass die Schwankung fast bei 100 % des lokalen Minimums 40 liegt.

Die in Fig. 2 dargestellten Minimumswerte sind aufgrund der Messkonstellation so stark schwankend, dass hier eine zuverlässige Auswertung durch einen einfachen Leerabgleich bzw. Subtrahieren der Signalwerte nur zu einem sehr ungenauen Messwert führt. Eine Messung der Minima in einem festen Maschinentakt ist ebenfalls nicht möglich.

Um den Einfluss der einzelnen auftretenden Variationen der Minima-Werte 36 - 48 zu minimieren, wird erfindungsgemäß vorgeschlagen, einen gleitenden Mittelwert über die Signalminima zu bilden und diese für die Differenzbildung zu verwenden. Es wird daher für die Differenzbildung ein gleitender Mittelwert $A_{min\_AV}$ über die Signalbildung verwendet. Der gleitende Mittelwert lautet:

$$A_{min\_AV} = (A_{min\_1} + A_{min\_2} + \ldots + A_{min\_N})/N$$

wobei $\{A_{min\_i}\}$ die letzten lokalen Minima für die Resonanzfrequenzverschiebung A bezeichnen. Der so gebildete gleitende Mittelwert wird zur Differenzbildung eingesetzt, so dass als Messgröße A folgender Wert verwendet wird:

$$A = A_{\_Kern} - A_{min\_AV},$$

wobei $A_{\_Kern}$ der für den Windelkern gemessene A-Wert ist. Mit den B-Werten wird analog verfahren.

Neben der vorstehend aufgeführten arithmetischen Mittelwertbildung für die A-Werte können auch andere Mittelwertbildungen verwendet werden. Insbesondere kann es von Interesse sein, auch gewichtete Mittelwerte zu verwenden, bei denen länger zurückliegende Messwerte mit einem geringeren Gewicht in die Messung eingehen, als gerade erfasste Minimumswerte.

Die mit Hilfe der Mikrowellenresonatoren bestimmten Messgrößen A, B lassen sich in unterschiedlicher Hinsicht auswerten. Da bei der Herstellung von Windeln Superabsorber als sehr hydrophiles Material eingesetzt werden, ist insbesondere auch die Bestimmung der Feuchte bzw. eines Feuchteprofils für den Windelkern von besonderem Interesse. Auch ist von Interesse, die Gesamtmasse und ein Dichteprofil für die Windeln aus den Messgrößen A, B zu bestimmen.

Die Gesamtmasse der Windelkerne erhält man durch Aufsummieren oder Integrieren der Messsignale A, B über den jeweiligen Windelkern. Teilt man den Integralwert durch die Anzahl der Einzelmessungen M, so lässt sich die Masse des Windelkerns mit folgender Kalibrationsgleichung bestimmen:

$$Masse_{Windelkern} = a_1 \cdot Int(A)/M + a_2 \cdot Int(B)/M + a_3,$$

wobei hier die Funktion "Int" für das Integral oder die Summe der Werte steht und M die Anzahl der Messung über dem Windelkern beschreibt. Die Parameter $a_1$, $a_2$ und $a_3$ können beim Kalibrieren für den weiteren Vorgang festgelegt werden. Die in der Windel enthaltene Feuchte kann durch Auswertung der Feuchtewerte mit B/A entsprechend erfolgen. Die Parameter $a_1$, $a_2$, $a_3$ sind abhängig von der Länge des Windelkerns und unabhängig von der Transportgeschwindigkeit.

Bei der Bestimmung der Gesamtmasse der Windelkerne ist es grundsätzlich auch möglich, die Masse abhängig von dem Wert des Integrals der Mikrowellenmesswerte zu bestimmen. Dieser Ausdruck lautet:

$$Masse' = a_1' \cdot Int(A) + a_2' \cdot Int(B) + a_3',$$

wobei $a_1'$, $a_2'$ und $a_3'$ Parameter sind, die über eine Kalibration bestimmt werden. Interessant ist hier, dass die Kalibration jeweils nur für eine Produktionsgeschwindigkeit durchgeführt werden kann, da die Werte der Integrale abhängig von der Geschwindigkeit ist. Theoretisch ist es möglich, die Geschwindigkeit, mit der die Windel durch den Sensor bewegt wird, mit in der Kalibrationsgleichung zu berücksichtigen.

In der Praxis hat sich herausgestellt, dass der Ansatz, den Mittelwert der Integralen (Int(A)/M, Int(B)/M) zu betrachten, die genauere Werte liefert, da der Mittelwert der Integrale nur von der Länge der Windelkerne abhängt, die sehr wenig schwankt.

Eine weitere Besonderheit bei der Messung mit Mikrowellenresonatoren für absorbierende Hygieneartikel besteht darin, dass das bahnförmige Material unter Umständen eine große Breite besitzen kann. Bevorzugt werden für die Messung an bahnförmigem Material breite Gabelsensoren eingesetzt, wie sie beispielsweise im Textilbereich aus EP 1 316 630 B1 bekannt ist. Diese Gabelsensoren bestehen im Wesentlichen aus einem entlang seiner Längsachse halbierten Zylinder, durch den das bahnförmige Material geführt wird. Gabelsensoren besitzen eine große Feldhomogenität in Zylinderrichtung, also senkrecht zur Transportrichtung der zu messenden Bahn. Daher ist es möglich, Dichte- und Masseprofile quer zur Bewegungsrichtung genau zu messen, wobei der Sensor einen Streifen in Bewegungsrichtung integrierend misst.

Zur Erreichung der erwähnten Feldhomogenität wird in einem Gabelsensor die sogenannte Grundmode E010 betrieben. Bezeichnet man mit d den Durchmesser eines zylindrischen Gabelsensors und 1 seine Länge, so gilt für d/1 → 0, dass sich die Resonanzfrequenz $f_0$ der Mode E011 zunehmend der Grundmode E010 annähert. Bei dieser Annäherung ist die Grundmode in der Praxis nicht mehr für eine Messung verwendbar, da stets Effekte und Beiträge der mitangeregten Mode E011 ihre Wirkung zeigen. Somit ist aus physikalischen Gründen die Länge von Gabelsensoren begrenzt und kann nicht beliebig skaliert oder verlängert werden. So lassen sich für Frequenzen von 2 - 3 GHz beispielsweise Gabelsensoren bis höchstens zu einer Länge von ca. 20 cm betreiben. Eine solche Messbreite kann für Hygieneartikel, beispielsweise bei Windeln für Erwachsene, zu wenig sein.

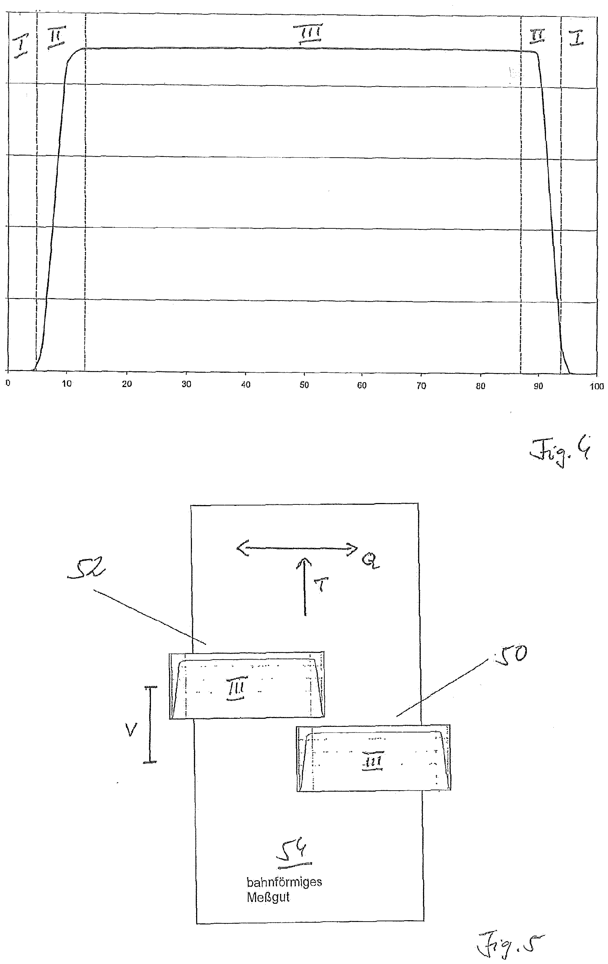

Fig. 4 zeigt die Feldstärkeverteilung in einem Gabelsensor in Richtung der Zylinderachse. Es können drei Zonen unterschieden werden:

I. Ein Feldstärkebereich außerhalb des Resonanzraums, in dem die Feldstärke exponentiell abnimmt.
II. Ein inhomogener Feldstärkebereich an Boden und Deckel der Zylinderstruktur, und
III. ein homogener Feldstärkebereich, der zur Messung von Masse und Dichte besonders gut geeignet ist.

Eine in Fig. 5 dargestellte, versetzte Anordnung der Gabelresonatoren 50, 52 erlaubt es, mit dem homogenen Feldbereichen III der beiden Gabelresonatoren 50, 52 das gesamte bahnförmige Messgut 54 zu erfassen. Die homogenen Messbereiche III der Gabelresonatoren 50, 52 sind in Querrichtung Q und in Transportrichtung T zueinander versetzt.

Der zwischen den Gabelresonatoren 50 und 52 auftretende Versatz V in Transportrichtung kann bei der Auswertung der Messsignale durch einen zeitlichen Versatz berücksichtigt werden.

Bei der Verwendung der Sensoren 50, 52 wird die Masse pro Längeneinheit durch die folgenden vier Messwerte bestimmt:

$$A_1, B_1, A_2, B_2,$$

wobei A die Resonanzfrequenzverschiebung und B die Verbreiterung der Resonanz bezeichnet, die Indizes beziehen sich auf die Resonatoren 50 und 52.

Die Masse pro Längeneinheit m in beispielsweise g/cm ist durch folgende Beziehung bestimmbar:

$$m = a_1 \cdot A_1 + a_2 \cdot A_2 + a_3 \cdot B_1 + a_4 \cdot B_2 + a_5,$$

wobei $a_i$ die Kalibrationskoeffizienten bezeichnet.

Gleichzeitig kann die Feuchte des Messguts u in % gemessen werden. Zur dichteunabhängigen Feuchtemessung wird der Quotient der Mikrowellenmesswerte verwendet. Man definiert, wie allgemein üblich, $\Phi = B/A$ oder $\Phi = \arctan(B/A)$. Die Feuchte des Messguts u in % wird unter Verwendung der Mikrowellenfeuchtewerte der beiden Resonatoren bestimmt. Es sei $\Phi_1 = \mathrm{actan}(B_1/A_1)$ und $\Phi_2 = \arctan(B_2/A_2)$ für die Mikrowellenresonatoren 50, 52. Der Feuchtewert u bestimmt sich dann:

$$u = b_1 \cdot \Phi_1 + b_2 \cdot \Phi_2 + b_3,$$

wobei $b_i$ die Kalibrationskoeffizienten bezeichnet.

Aufgrund von Fertigungstoleranzen für die Gabelsensoren ergeben die Messungen in beiden Gabelresonatoren für das gleiche Produkt leicht unterschiedliche Messwerte für die Messgrößen A und B. Durch Testmessungen an einem homogenen Material können die beiden Messgrößen der Resonatoren 50, 52 in Beziehung zueinander gesetzt werden. Ein Versatz zwischen den Resonatoren kann für ein homogenes Material entfallen. Ein Ansatz für die Auswertung der Messsignale kann daher sein:

$$A_1 = c_1 \cdot A_2,$$

$$B_1 = c_2 \cdot B_2,$$

wobei $c_1$ und $c_2$ vorbestimmte Koeffizienten sind. Der Vorteil, die Messgrößen der beiden Mikrowellenresonatoren vorab in ein konstantes Verhältnis $c_1$, $c_2$ zueinander zu setzen, besteht darin, dass für die Masse- und Feuchtewerte weniger Kalibrationskoeffizienten $a_i$ bestimmt werden müssen.

Grundsätzlich kann die vorbeschriebene Anordnung der Mikrowellenresonatoren 50, 52 auch auf mehr als zwei Mikrowellenresonatoren erweitert werden, um die zu messende Bahn mit einer möglichst homogenen Feldverteilung zu vermessen.

**Patentansprüche**

1. Vorrichtung zur Messung von absorbierenden Hygieneartikeln, die eine fortlaufende Bahn (54) mit voneinander beabstandeten Saugkörpern aufweist, sowie zwei oder mehr Mikrowellenresonatoren (50, 52), die dazu ausgebildet sind, mit Hilfe einer Resonanzfrequenzverschiebung (A) und einer Resonanzfrequenzverbreiterung (B) Feuchte und/oder Dichte der Saugkörper zu erfassen, wobei die Bahn (54) dazu ausgebildet ist, durch die Mikrowellenresonatoren transportiert zu werden, wobei die zwei Mikrowellenresonatoren (50, 52) dazu ausgebildet sind,

   fortlaufend zwei Mikrowellengrößen (A, B) zu erfassen, wobei die zwei Mikrowellenresonatoren, bezogen auf eine Transportrichtung der Bahn zueinander in Quer- und Transportrichtung (Q, T) versetzt angeordnet sind, um die Bahn (54) in ihrer gesamten Breite zu vermessen, **dadurch gekennzeichnet, dass** jeder der Mikrowellenresonatoren (50,52) einen Messbereich mit einem homogenen Feldverlauf (III) besitzt und die mehreren Mikrowellenresonatoren (50, 52) derart quer zur Transportrichtung positioniert sind, dass die gesamte Breite der Bahn (54) von einem Messbereich mit homogenem Feldverlauf (III) überdeckt ist.

2. Vorrichtung nach Anspruch 1, **dadurch gekennzeichnet, dass** die Messwerte der verschiedenen Mikrowellenresonatoren (50, 52) um den Versatz (V) der Mikrowellenresonatoren in Transportrichtung korrigiert werden.

3. Vorrichtung nach Anspruch 1 oder 2 , **dadurch gekennzeichnet, dass** mindestens ein Gabelsensor zur Messung der Bahn vorgesehen ist.

4. Vorrichtung nach einem der Ansprüche 1 bis 3, **dadurch gekennzeichnet, dass** die Messbereiche der Mikrowellenresonatoren (50, 52) in Querrichtung einander überlappen.

5. Vorrichtung nach einem der Ansprüche 1 bis 4, **dadurch gekennzeichnet, dass** in Transportrichtung der Abstand zwischen zwei Saugkörpern kleiner als ein jeweiliger Durchmesser der Mikrowellenresonatoren ist und die Signalminima der Mikrowellengrößen anstatt eines Leerabgleichs ausgewertet werden.

6. Verfahren zur Messung von absorbierenden Hygieneartikeln, die auf eine fortlaufende Bahn (54) mit voneinander beabstandeten Saugkörpern aufgebracht werden, wobei zwei oder mehr Mikrowellenresonatoren (50, 52) dazu ausgebildet sind, mit Hilfe einer Resonanzfrequenzverschiebung (A) und einer Resonanzfrequenzverbreiterung (B) Feuchte und/oder Dichte der Saugkörper zu erfassen, wobei die Bahn dazu ausgebildet ist, durch die Mikrowellenresonatoren transportiert zu werden,, **dadurch gekennzeichnet, dass** mindestens zwei Mikrowellenresonatoren (50,52) dazu ausgebildet sind,, fortlaufend zwei Mikrowellenmessgrößen (A, B) zu erfassen, wobei die zwei Mikrowellenresonatoren die bezogen auf eine Transportrichtung der Bahn (54) zueinander in Quer- und Transportrichtung (Q, T) versetzt angeordnet sind, um die Bahn in ihrer gesamten Breite zu vermessen, **dadurch gekennzeichnet, dass** jeder der Mikrowellenresonatoren einen Messbereich mit einem homogenen Feldverlauf (III) besitzt und die mehreren Mikrowellenresonatoren derart quer zur Transportrichtung positioniert sind, dass die gesamte Breite der Bahn von einem Messbereich mit homogenem Feldverlauf (III) überdeckt ist.

7. Verfahren nach Anspruch 6, **dadurch gekennzeichnet, dass** die gemittelten Messwerte der einzelnen Mikrowellenresonatoren (50, 52) um den Versatz (V) der Mikrowellenresonatoren in Transportrichtung korrigiert werden.

8. Verfahren nach einem der Ansprüche 6 oder 7, **dadurch gekennzeichnet, dass** die Masse eines Saugkörpers über die aufsummierten Messwerte der beiden Messgrößen bestimmt wird.

9. Verfahren nach einem der Ansprüche 6 bis 8, **dadurch gekennzeichnet, dass** die Feuchte eines Saugkörpers über aufsummierte Messwerte der beiden Messgrößen bestimmt wird.

10. Verfahren nach Anspruch 8 oder 9, **dadurch gekennzeichnet, dass** die aufsummierten Messwerte durch die Anzahl der Summanden dividiert werden.

11. Verfahren nach Anspruch 6 bis 10, **dadurch gekennzeichnet, dass** die Messbereiche der Mikrowellenresonatoren in Querrichtung einander überlappen.

12. Verfahren nach einem der Ansprüche 6 bis 11, **dadurch gekennzeichnet, dass** in Transportrichtung der Abstand zwischen zwei Saugkörpern kleiner als der jeweilige Durchmesser der Mikrowellenresonatoren ist und die Signalminima der Mikrowellen anstatt eines Leerabgleichs ausgewertet werden.

## Claims

1. A device for measuring absorbent hygiene articles, which has a continuous sheet (54) with absorbent bodies spaced apart from each other,

   and two or more microwave resonators (50, 52) which are designed to detect moisture and/or density of the absorbent bodies based on a resonant frequency shift (A) and a resonant frequency distribution (B), wherein the sheet (54) is designed to be transported through the microwave resonators,
   wherein the two microwave resonators (50, 52) are designed to continuously detect two microwave variables (A, B), wherein the two microwave resonators, in relation to a transport direction of the sheet, are arranged offset from each other in the transverse and transport directions (Q, T) in order to measure the entire width of the sheet (54),
   **characterized in that** each of the microwave resonators (50, 52) has a measurement region with a homogenous field profile (III) and the multiple microwave resonators (50, 52) are positioned transversely to the transport direction such that the entire width of the sheet (54) is covered by a measurement region with a homogeneous field profile (III).

2. The device according to claim 1, **characterized in that** the measured values of the various microwave resonators (50, 52) are corrected by the offset (V) of the microwave resonators in the transport direction.

3. The device according to claim 1 or 2, **characterized in that** at least one fork sensor is provided for measuring the sheet.

4. The device according to one of claims 1 to 3, **characterized in that** the measurement regions of the microwave resonators (50, 52) overlap each other in the transverse direction.

5. The device according to one of claims 1 to 4, **characterized in that** the distance between two absorbent bodies in the transport direction is smaller than a respective diameter of the microwave resonators, and the signal minima of the microwave variables is evaluated instead of an empty adjustment.

6. A method for measuring absorbent hygiene articles which are applied to a continuous sheet (54) with absorbent bodies spaced apart from each other, wherein two or more microwave resonators (50, 52) are designed to detect moisture and/or density of the absorbent bodies based on a resonant frequency shift (A) and a resonant frequency distribution (B), wherein the sheet is designed to be transported through the microwave resonators, **characterized in that** at least two microwave resonators (50, 52) are designed to continuously detect two microwave measured variables (A, B), wherein the two microwave resonators which, in relation to a transport direction of the sheet (54), are arranged offset from each other in the transverse and transport directions (Q, T) in order to measure the entire width of the sheet, **characterized in that** each of the microwave resonators has a measurement region with a homogeneous field profile (III) and the multiple microwave resonators are positioned transversely to the transport direction such that the entire width of the sheet is covered by a measurement region with a homogeneous field profile (III).

7. The method according to claim 6, **characterized in that** the averaged measured values of the individual microwave resonators (50, 52) are corrected by the offset (V) of the microwave resonators in the transport direction.

8. The method according to one of claims 6 or 7, **characterized in that** the mass of an absorbent body is determined by adding up the measured values of the two measured variables.

9. The method according to one of claims 6 to 8, **characterized in that** the moisture of an absorbent body is determined by adding up the measured values of the two measured variables.

10. The method according to claim 8 or 9, **characterized in that** the added measured values are divided by the number of summands.

11. The method according to one of claims 6 to 10, **characterized in that** the measurement regions of the microwave resonators overlap each other in the transverse direction.

12. The method according to one of claims 6 to 11, **characterized in that** the distance between two absorbent bodies in the transport direction is smaller than the respective diameter of the microwave resonators, and the signal minima of the microwaves are evaluated instead of an empty adjustment.


**Revendications**

1. Dispositif de mesure d'articles d'hygiène absorbants, lequel présente une voie continue (54) avec des corps d'aspiration espacés les uns des autres, et

   deux ou plusieurs résonateurs à microondes (50, 52) conçus pour détecter l'humidité et/ou la densité des corps d'aspiration à l'aide d'un décalage de fréquence de résonateur (A) et d'un élargissement de fréquence de résonateur (B), la voie (54) étant conçue pour être transportée à travers les résonateurs à microondes,
   dans lequel les deux résonateurs à microondes (50, 52) sont conçus pour détecter en continu deux grandeurs de microondes (A, B), dans lequel les deux résonateurs à microondes sont disposés de façon décalée l'un par rapport à l'autre dans la direction transversale et la direction de transport (Q, T) relativement à une direction de transport de la voie, afin de mesurer la voie (54) dans toute sa largeur, **caractérisé en ce que**
   chacun des résonateurs à microondes (50, 52) possède une plage de mesure avec une évolution de champ homogène (III) et la pluralité de résonateurs à microondes (50, 52) sont positionnés de telle façon transversalement à la direction de transport, que toute la largeur de la voie (54) est couverte par une plage de mesure avec une évolution de champ homogène (III).

2. Dispositif selon la revendication 1, **caractérisé en ce que** les grandeurs de mesure des différents résonateurs à microondes (50, 52) sont corrigées quant au décalage (V) des résonateurs à microondes dans la direction de transport.

3. Dispositif selon la revendication 1 ou 2, **caractérisé en ce qu'**il est prévu au moins un capteur à fourche pour la mesure de la voie.

4. Dispositif selon l'une des revendications 1 à 3, **caractérisé en ce que** les plages de mesure des résonateurs à microondes (50, 52) se chevauchent dans la direction transversale.

5. Dispositif selon l'une des revendications 1 à 4, **caractérisé en ce que** dans la direction de transport, l'espacement entre deux corps d'aspiration est plus petit qu'un diamètre respectif des résonateurs à microondes et les minima du signal des grandeurs de microondes sont évalués à la place d'un réglage vide.

6. Procédé de mesure d'articles d'hygiène absorbants, lesquels sont appliqués sur une voie continue (54) avec des corps d'aspiration espacés les uns des autres, dans lequel deux ou plusieurs résonateurs à microondes (50, 52) sont conçus pour détecter l'humidité et/ou la densité des corps d'aspiration à l'aide d'un décalage de fréquence de résonateur (A) et d'un élargissement de fréquence de résonateur (B), la voie étant conçue pour être transportée à travers les résonateurs à microondes,
**caractérisé en ce qu'**au moins deux résonateurs à microondes (50, 52) sont conçus pour détecter en continu deux grandeurs de microondes (A, B), dans lequel les deux résonateurs à microondes sont disposés de façon décalée l'un par rapport à l'autre dans la direction transversale et la direction de transport (Q, T) relativement à une direction de transport de la voie, afin de mesurer la voie dans toute sa largeur, **caractérisé en ce que** chacun des résonateurs à microondes possède une plage de mesure avec une évolution de champ homogène (III) et la pluralité de résonateurs à microondes sont positionnés de telle façon transversalement à la direction de transport, que toute la largeur de la voie est couverte par une plage de mesure avec une évolution de champ homogène (III).

7.  Procédé selon la revendication 6, **caractérisé en ce que** les valeurs de mesure moyennes des différents résonateurs à microondes (50, 52) sont corrigées quant au décalage (V) des résonateurs à microondes dans la direction de transport.

8.  Procédé selon l'une des revendications 6 ou 7, **caractérisé en ce que** la masse d'un corps d'aspiration est déterminée par la somme des valeurs de mesure des deux grandeurs de mesure.

9.  Procédé selon l'une des revendications 6 à 8, **caractérisé en ce que** l'humidité d'un corps d'aspiration est déterminée par la somme de valeurs de mesure des deux grandeurs de mesure.

10. Procédé selon la revendication 8 ou 9, **caractérisé en ce que** la somme de valeurs de mesure est divisée par le nombre d'éléments additionnés.

11. Procédé selon la revendication 6 à 10, **caractérisé en ce que** les plages de mesure des résonateurs à microondes se chevauchent entre elles dans la direction transversale.

12. Procédé selon l'une des revendications 6 à 11, **caractérisé en ce que** dans la direction de transport, l'espacement entre deux corps d'aspiration est plus petit que le diamètre respectif des résonateurs à microondes et les minima du signal des microondes sont évalués à la place d'un réglage vide.

Fig. 1

Fig. 2

Fig. 3

Fig. 4

bahnförmiges
Meßgut

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1327876 B1
- WO 2014170796 A1
- EP 1933132 A2
- US 2016051414 A1
- DE 102009004457 A1
- EP 1467191 A1
- EP 1316630 B1